# EUROPEAN PATENT SPECIFICATION

(11) **EP 2 989 138 B1**
(45) Date of publication and mention of the grant of the patent: **01.08.2018**
(21) Application number: 14788269.0
(22) Date of filing: 11.03.2014
(51) Int. Cl.: C08G 18/32, C07D 493/10, C08G 18/67, C08G 18/75, C08G 18/81, C09D 175/16

(54) **URETHANE ACRYLATES BASED ON 2,4,8,10-TETRAOXOSPIRO[5.5]UNDECANE-3,9-DIALKANOLS**
URETHANACRYLATE AUF BASIS VON 2,4,8,10-TETRAOXOSPIRO-[5,5-]UNDECAN-3,9-DIALKANOLEN
ACRYLATES D'URÉTHANE DÉRIVÉS DE 2,4,8,10-TÉTRAOXOSPIRO[5.5]UNDÉCANE-3,9-DIALCANOLS

(30) Priority: 22.04.2013 SE 1300292
(43) Date of publication of application: 02.03.2016
(73) Proprietor: PERSTORP AB, 284 80 Perstorp (SE)
(72) Inventor: JAMES, David, 224 67 Lund (SE); ZELLNER, Linda, 263 63 Viken (SE)
(74) Representative: Hoffmann Eitle
(86) International application number: PCT/SE2014/000024
(87) International publication number: WO 2014/175802

(56) References cited:
- JP-A- S5 938 223
- JP-A- S5 938 223
- US-A- 4 587 352
- US-A- 4 587 352
- US-A1- 2008 200 582
- DATABASE WPI Week 198231 Thomson Scientific, London, GB; AN 1982-64785E XP002762918, & JP S57 102891 A (MITSUBISHI GAS CHEM IND CO LTD) 26 June 1982 (1982-06-26)

## Description

The present invention refers to a novel urethane acrylate being a reaction product of at least a diol component, a component having acrylic unsaturation and an isocyanate component, wherein said diol component comprises at least one alkoxylated 2,4,8,10-tetraoxaspiro[5.5]-undecane-3,9-dialkanol.

Radiation, such as UV-curing coatings for industrial applications has been rising constantly for many years. Less productive and/or less environmentally friendly systems have gradually been replaced by radiation technology, and an ever increasing number of new applications is being found for radiation curing coatings. Radiation curable polyurethane acrylates have been well known for more than two decades. The most common are coatings based on urethane acrylate or methacrylate oligomers, which represent a major class of coatings widely used in industry.

Such coatings are used for protection purposes as well as applications such as heat and/or radiation curing coatings, inks and adhesives. Cured coatings typically yield films with for instance a good combination of toughness and elasticity. The urethane acrylate is the main part of the composition, thus determining the properties of both the liquid and the cured product.

There is, however, despite the large amount of commercially available urethane acrylates and derivatives thereof, due to for instance environmental concerns and legislation and/or new application areas, a substantial and ever growing demand for novel, safer, more efficient and/or more versatile urethane acrylates. A major search, for safer urethane acrylates, is directed to suitable replacements for presently used urethane acrylates based on bisphenols and alkoxylated bisphenols, such as the frequently used ethoxylated bisphenol A. Bisphenols and derivatives thereof are known to be endocrine disrupters that mimic for instance oestrogen and are associated with irritation to the respiratory system, risks of serious damage to the eyes and neural system, risk of sensitisation by skin contact and risk of impaired fertility. The hazardous effects of inadvertent exposure to bisphenols and bisphenol releasing chemicals in professionals and in the general population should thus be avoided.

US 4 587 352 A discloses a urethane (meth)acrylate compound which can be obtained by allowing (meth)acrylate to react with a reaction product of diisocyanate with spiro glycol.

JP S57 102891 discloses a polyether diol in form of an alkoxylated spiroglycol for making polyurethanes, which can be used in elastomers, adhesives and coatings, and for making photosetting monomers.

Hydrogenated bisphenols have been suggested as replacement for corresponding bisphenols. There are, however, significant drawbacks associated with the use of for instance hydrogenated bisphenol A. Drawbacks include for instance the fact that hydrogenated bisphenol A is a high melting solid and thus difficult to handle in a plant environment and the fact that reactions between liquids and high melting solids are difficult to moderate. It would accordingly be desirable to have a diol which provide the property advantages of a high melting point diol with reduced processing problems. A further disadvantage is that production of hydrogenated bisphenols means handling of and contact with the hazardous bisphenols.

An object of the present invention is to provide a novel urethane acrylate having at least one acrylic, methacrylic and/or methylacrylic double bond. A further object is to provide a suitable replacement for urethane acrylates involving handling of hazardous and/or restricted compounds such as bisphenols, bisphenol releasing substances and especially compounds derived from bisphenols, such as alkoxylated bisphenols.

It has now quite unexpectedly been found that an alkoxylated 2,4,8,10-tetraoxaspiro[5.5]-undecane-3,9-dialkanol is a versatile compound in most applications involving urethane acylates. The novel urethane acrylate of the present invention can thus advantageously replace urethane acrylates based on alkoxylated bisphenols, as well as bisphenols, as a product being safer to handle, safer to produce and/or being environmentally friendlier.

The novel urethane acrylate of the present invention is a reaction product of a raw material composition comprising at least (A) a diol component, (B) a component having acrylic unsaturation and (C) an isocyanate component. Said component (A) comprises at least one alkoxylated 2,4,8,10-tetraoxaspiro[5.5]undecane-3,9-dialkanol of formula 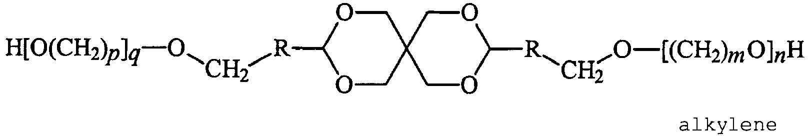 wherein each substituent R individually is a linear or branched C₁-C₈ alkylene group and *m, n, p* and *q* individually are integers each being at least 1, said component (B) comprises, in embodiments thereof, at least one hydroxyalkyl acrylate, hydroxyalkyl methacrylate and/or hydroxyalkyl methylacrylate, whereby hydroxyalkyl preferably is hydroxy-C₁-C₁₂-alkyl, which can be exemplified by hydroxyethyl (meth)acrylate, hydroxypropyl (meth)acrylate, trimethylolpropane di(meth)acrylate and pentaerythritol tri(meth)acrylate, and/or comprises acrylic acid, methacrylic acid and/or a methylacrylic acid, such as crotonic or isocrotonic acid, or a corresponding alkyl ester thereof, and wherein said component (C) comprises at least one di or polyisocyanate or a derivative thereof.

In preferred embodiments of said alkoxylated 2,4,8,10-tetraoxaspiro[5.5]undecane-3,9--dialkanol, *m* and *p,* in above formula, are independently integers each being between 1 and 6, such as between 1 and 4 and *n* and *q* are independently integers each being between 1 and 10, such as between 1 and 8, between 1 and 6 or between 1 and 4. Substituent R in above formula is in likewise preferred embodiments an alkyl group of formula

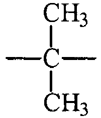

The most preferred embodiments of said alkoxylated 2,4,8,10-tetraoxaspiro[5.5]-undecane-3,9-dialkanol include alkoxylated, such as ethoxylated, propoxylated and/or butoxylated 2,4,8,10-tetraoxaspiro[5.5]undecane-3,9-diethanol.

The especially preferred embodiments of said alkoxylated 2,4,8,10-tetraoxaspiro[5.5]-undecane-3,9-diethanol (pentaerythritol spiroglycol) is an ethoxylated and/or propoxylated 2,4,8,10-tetraoxaspiro[5.5]undecane-3,9-diethanol having 1-10, such as 1-6, 1-5, 1-4, 1-3, 1-2 or most preferably 3-6 ethoxy and/or propoxy units / hydroxyl group.

Said component (A) comprises, in preferred embodiments of the novel urethane acrylate according to the present invention, 50-100%, such as 70-100% or 80-100%, by weight of said alkoxylated 2,4,8,10-tetraoxaspiro[5.5]undecane-3,9-dialkanol, such as said ethoxylated, propoxylated and/or butoxylated 2,4,8,10-tetraoxaspiro[5.5]undecane-3,9-diethanol, optionally in combination with one or more other hydroxyfunctional compound(s) known in the art. In likewise preferred embodiments, said component (B) comprises 50-100% by weight of said at least one hydroxyalkyl acrylate, hydroxyalkyl methacrylate and/or hydroxyalkyl methylacrylate and/or 50-100% by weight of said acrylic acid, methacrylic acid and/or a methylacrylic acid and/or an alkyl ester thereof.

Said di or polyisocyanate is preferably and advantageously selected from the group consisting of toluene diisocyanate, diphenyl methane diisocyanate, hexamethylene diisocyanate, isophorone diisocyanate, tetramethylxylene diisocyanate, dicyclohexyl methane diisocyanate, furan diisocyanate, tetrahydrofuran diisocyanate, cyclohexylene diisocyanate, xylene diisocyanate, naphthalene diisocyanate, phenylene diisocyanate, nonane triisocyanate and/or triphenyl methane triisocyanate.

Said raw material composition may, in addition to said components (A), (B) and (C), comprise one or more carboxylic acids, such as octanoic acid, 2-ethylhexanoic acid, 2-propylheptanoic acid, cecanoic acid and/or trimethylhexanoic acid.

Without further elaboration, it is believed that one skilled in the art can, using the preceding description, utilise the present invention to its fullest extent. The following preferred specific embodiments are, therefore, to be construed as merely illustrative and not limitative. In the following Examples 1 and 2 illustrate an embodiment of the present invention and Examples 1 and 3 a comparative product based on bisphenol A ethoxylate. Example 4 refer to curing of the products yielded in Examples 2 and 3. Table 1 returns properties measured on products yielded in Examples 2 and 3.

### Example 1

An adduct between isophorone diisocyanate and hydroxyethyl acrylate was produced for use in productions of urethane acrylates as disclosed in Examples 2 and 3 (comparative).

175.0 parts by weight of isophorone diisocyanate and 0.6 parts by weight of methoxy phenol, as inhibitor, were charged to a reaction vessel, equipped with a heating device, temperature control, stirrer, air inlet and a cooler. 86.8 parts by weight of hydroxyethyl acrylate was successively during 10 minutes charged and 0.03 part by weight of dibutyltinlaurate as catalyst was subsequently charged to the reaction mixture. The temperature was now slowly raised to 60-70°C and the reaction was allowed to continue until the theoretical remaining NCO value was reached. Yielded reaction product was finally cooled to room temperature.

### Example 2

54.5 parts by weight of an ethoxylated 2,4,8,10-tetraoxaspiro[5.5]undecane-3,9-diethanol, having an average of 4-5 ethylene oxide units / hydroxyl group (pentaerythritol spiroglycol ethoxylate, Perstorp Specialty Chemicals AB), 0.2 parts by weight of methoxy phenol as inhibitor and 133.0 parts by weight of toluene as azeotropic solvent were charged to a reaction vessel, equipped with a heating device, temperature control, stirrer, air inlet and reflux. 100.0 parts by weight of the adduct yielded in Example 1 was now slowly charged to the reaction mixture followed by charging of 0.03 parts by weight of dibutyltinlaurate as catalyst. The temperature was rapidly raised to 65°C and maintained until the NCO value was close to 0. Toluene was now evaporated and remaining reaction product was cooled to room temperature. Properties measured on obtained product are given in Table 1.

### Example 3 (comparative)

Example 2 was repeated with the difference that 52.4 parts by weight of a bisphenol A ethoxylate having an average of 4 ethoxy units / phenolic hydroxyl group (Sigma Aldrich Co) was charged instead of 54.5 parts by weight of the ethoxylated 2,4,8,10-tetraoxaspiro-[5.5]undecane-3,9-diethanol. Properties measured on obtained product are given in Table 1.

### Example 4

The products yielded in Examples 2 and 3 (comparative) were, after addition of 1.0 part by weight of Irgacure 500 (Photoinitiator, Ciba Specialty Chemicals Inc.), coated on glass and steel panels at a dry filmthickness of 40 µm and cured.

The coated panels were first placed in an oven for 30 minutes at 50°C, cooled to room temperature and subsequently UV cured. UV curing was performed by means of a UV-lamp of 80W/cm² and at a belt speed of 20 m/min. The lacquers were allowed to pass the UV-lamp 4 times. The samples were after curing conditioned for 24 hours at 23 ± 2°C and 50 ± 5% relative humidity followed by measuring of filmhardness, flexibility and gloss.

### Recorded results:

| | Example 2 | Example 3 |
|---|---|---|
| Hardness, König sees. | 214 | 200 |
| Erichsen flexibility, mm | 0.4 | 2.6 |
| Gloss at 60° | 89 | 94 |

**Table 2**

| | **Example 2** | **Example 3** |
|---|---|---|
| Hydroxyl value, mg KOH/g | 4 | 5 |
| Acid value, mg KOH/g | 0.1 | 0.3 |
| Molecular weight Mw, g/mol | 2 200 | 2 400 |
| Acrylic concentration IR | 1.40 | 1.42 |
| Polydispersity | 1.4 | 1.4 |

## Claims

1. A urethane acrylate being a reaction product of a raw material composition comprising at least (A) a diol component, (B) a component having acrylic unsaturation and (C) an isocyanate component **characterised in, that** said component (A) comprises at least one alkoxylated 2,4,8,10-tetraoxaspiro[5.5]undecane-3,9-dialkanol of formula 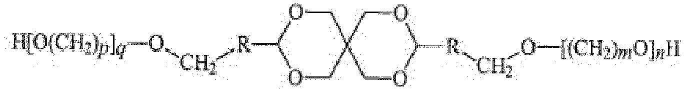 wherein each substituent R individually is a linear or branched C₁-C₈ alkylene group and *m, n, p* and *q* individually are integers each being at least 1, that said component (B) comprises at least one hydroxyalkyl acrylate, hydroxyalkyl methacrylate and/or hydroxyalkyl methylacrylate, and/or comprises acrylic acid, methacrylic acid and/or a methylacrylic acid and/or a corresponding alkyl ester of a said acrylic acid, and that said component (C) comprises at least one di or polyisocyanate and/or a derivative thereof.

2. The urethane acrylate according to Claim 1 **characterised in, that** said hydroxyalkyl is hydroxy-C₁-C₁₂-alkyl.

3. The urethane acrylate according to Claim 1 or 2 **characterised in, that** said component (B) comprises hydroxyethyl(meth)acrylate, hydroxypropyl (meth)acrylate, trimethylolpropane di(meth)acrylate and/or pentaerythritol tri(meth)acrylate.

4. The urethane acrylate according to any of the Claims 1-3 **characterised in, that** *m* and *p* independently are integers each being between 1 and 6.

5. The urethane acrylate according to any of the Claims 1-3 **characterised in, that** *m* and *p* independently are integers each being between 1 and 4.

6. The urethane acrylate according to any of the Claims 1-5 **characterised in, that** *n* and *q* independently are integers each being between 1 and 10.

7. The urethane acrylate according to any of the Claims 1-5 **characterised in, that** *n* and *q* independently are integers each being between 1 and 6.

8. The urethane acrylate according to any of the Claims 1-6 **characterised in, that** each substituent R is an alkyl group of formula

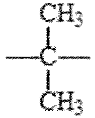

9. The urethane acrylate according to any of the Claims 1-8 **characterised in, that** said alkoxylated 2,4,8,10-tetraoxaspiro[5.5]undecane-3,9-dialkanol is an alkoxylated 2,4,8,10-tetraoxaspiro[5.5]undecane-3,9-diethanol.

10. The urethane acrylate according to any of the Claims 1-9 **characterised in, that** said alkoxylated is ethoxylated, propoxylated and/or butoxylated.

11. The urethane acrylate according to any of the Claims 1-10 **characterised in, that** said component (A) comprises 50-100% by weight of said alkoxylated 2,4,8,10-tetraoxaspiro[5.5]undecane-3,9-dialkanol.

12. The urethane acrylate according to any of the Claims 1-10 **characterised in, that** said component (A) comprises 70-100% by weight of said alkoxylated 2,4,8,10-tetraoxaspiro[5.5]undecane-3,9-dialkanol.

13. The urethane acrylate according to any of the Claims 1-10 **characterised in, that** said component (A) comprises 80-100% by weight of said alkoxylated 2,4,8,10-tetraoxaspiro[5.5]undecane-3,9-dialkanol.

14. The urethane acrylate according to Claims 1-13 **chara cterised in**, that said alkoxylated 2,4,8,10-tetraoxaspiro[5.5]undecane-3,9-diethanol is an ethoxylated and/or propoxylated 2,4,8,10-tetraoxaspiro[5.5]undecane-3,9-diethanol.

15. The urethane acrylate according to any of the Claims 1-14 **characterised in, that** said component (B) comprises 50-100% by weight of said hydroxyalkyl acrylate, hydroxyalkyl methacrylate and/or hydroxyalkyl methylacrylate.

16. The urethane acrylate according to any of the Claims 1-15 **characterised in, that** said component (B) comprises 50-100% by weight of said acrylic acid, methacrylic acid and/or a methylacrylic acid and/or an alkyl ester of a said acrylic acid.

17. The urethane acrylate according to any of the Claims 1-16 **characterised in, that** said di or polyisocyanate is toluene diisocyanate, diphenyl methane diisocyanate, hexamethylene diisocyanate, isophorone diisocyanate, tetramethylxylene diisocyanate, dicyclohexyl methane diisocyanate, furan diisocyanate, tetrahydrofuran diisocyanate, cyclohexylene diisocyanate, xylene diisocyanate, naphthalene diisocyanate, phenylene diisocyanate, nonane triisocyanate and/or triphenyl methane triisocyanate.

18. The urethane acrylate according to any of the Claims 1-17 **characterised in, that** said raw material composition additionally comprises at least one carboxylic acid.

19. The urethane acrylate according to Claim 18 **characterised in, that** said carboxylic acid is octanoic acid, 2-ethylhexanoic acid, 2-propylheptanoic acid, cecanoic acid and/or trimethylhexanoic acid.

20. The urethane acrylate according to any of the Claims 1-19 **characterised in, that** said component (A) comprises an ethoxylated 2,4,8,10-tetraoxaspiro[5.5]undecane-3,9-diethanol having an average of 3-6 ethoxy units / hydroxyl group, that said component (B) comprises hydroxyethyl acrylate and that said component (C) comprises isophorone diisocyanate.

## Patentansprüche

1. Urethanacrylat, das ein Reaktionsprodukt einer Rohstoffzusammensetzung ist, umfassend mindestens (A) eine Diolkomponente, (B) eine Komponente mit acrylischer Unsättigung und (C) eine Isocyanatkomponente, **dadurch gekennzeichnet,**
**dass** die Komponente (A) mindestens ein alkoxyliertes 2,4,8,10-Tetraoxaspiro[5.5]undecan-3,9-dialkanol der Formel 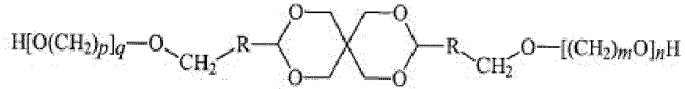 umfasst, worin jeder Substituent R unabhängig eine lineare oder verzweigte C₁-C₈-Alkylengruppe ist und *m, n, p* und *q* unabhängig Zahlen sind, die jeweils mindestens 1 sind,
**dass** die Komponente (B) mindestens eines aus Hydroxyalkylacrylat, Hydroxyalkylmethacrylat und/oder Hydroxyalkylmethylacrylat umfasst, und/oder Acrylsäure, Methacrylsäure und/oder eine Methylacrylsäure und/oder einen entsprechenden Alkylester der Acrylsäure umfasst, und
**dass** die Komponente (C) mindestens ein Di- oder Polyisocyanat und/oder ein Derivat davon umfasst.

2. Urethanacrylat gemäß Anspruch 1, **dadurch gekennzeichnet, dass** das Hydroxyalkyl Hydroxy-C₁-C₁₂-alkyl ist.

3. Urethanacrylat gemäß Anspruch 1 oder 2, **dadurch gekennzeichnet, dass** die Komponente (B) Hydroxyethyl(meth)acrylat, Hydroxypropyl(meth)acrylat, Trimethylolpropan-Di(meth)acrylat und/oder Pentaerythritol-Tri(meth)acrylat umfasst.

4. Urethanacrylat gemäß mindestens einem der Ansprüche 1-3, **dadurch gekennzeichnet, dass** *m* und *p* jeweils unabhängig Zahlen zwischen 1 und 6 sind.

5. Urethanacrylat gemäß mindestens einem der Ansprüche 1-3, **dadurch gekennzeichnet, dass** *m* und *p* jeweils unabhängig Zahlen zwischen 1 und 4 sind.

6. Urethanacrylat gemäß mindestens einem der Ansprüche 1-5, **dadurch gekennzeichnet, dass** *n* und *q* jeweils unabhängig Zahlen zwischen 1 und 10 sind.

7. Urethanacrylat gemäß mindestens einem der Ansprüche 1-5, **dadurch gekennzeichnet, dass** *n* und *q* jeweils unabhängig Zahlen zwischen 1 und 6 sind.

8. Urethanacrylat gemäß mindestens einem der Ansprüche 1-6, **dadurch gekennzeichnet, dass** jeder Substituent R eine Alkylgruppe der Formel 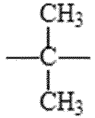 ist.

9. Urethanacrylat gemäß mindestens einem der Ansprüche 1-8, **dadurch gekennzeichnet, dass** das alkoxylierte 2,4,8,10-Tetraoxaspiro[5.5]undecan-3,9-dialkanol ein alkoxyliertes 2,4,8,10-Tetraoxaspiro[5.5]undecan-3,9-diethanol ist.

10. Urethanacrylat gemäß mindestens einem der Ansprüche 1-9, **dadurch gekennzeichnet, dass** alkoxyliert ethoxyliert, propoxyliert und/oder butoxyliert ist.

11. Urethanacrylat gemäß mindestens einem der Ansprüche 1-10, **dadurch gekennzeichnet, dass** die Komponente (A) 50-100 Gew.-% des alkoxylierten 2,4,8,10-Tetraoxaspiro[5.5]undecan-3,9-dialkanols umfasst.

12. Urethanacrylat gemäß mindestens einem der Ansprüche 1-10, **dadurch gekennzeichnet, dass** die Komponente (A) 70-100 Gew.-% des alkoxylierten 2,4,8,10-Tetraoxaspiro[5.5]undecan-3,9-dialkanols umfasst.

13. Urethanacrylat gemäß mindestens einem der Ansprüche 1-10, **dadurch gekennzeichnet, dass** die Komponente (A) 80-100 Gew.-% des alkoxylierten 2,4,8,10-Tetraoxaspiro[5.5]undecan-3,9-dialkanols umfasst.

14. Urethanacrylat gemäß mindestens einem der Ansprüche 1-13, **dadurch gekennzeichnet, dass** das alkoxylierte 2,4,8,10-Tetraoxaspiro[5.5]undecan-3,9-diethanol ein ethoxyliertes und/oder propoxyliertes 2,4,8,10-Tetraoxaspiro[5.5]undecan-3,9-diethanol ist.

15. Urethanacrylat gemäß mindestens einem der Ansprüche 1-14, **dadurch gekennzeichnet, dass** die Komponente (B) 50-100 Gew.-% des Hydroxyalkylacrylats, Hydroxyalkylmethacrylats und/oder Hydroxyalkylmethylacrylats umfasst.

16. Urethanacrylat gemäß mindestens einem der Ansprüche 1-15, **dadurch gekennzeichnet, dass** die Komponente (B) 50-100 Gew-% der Acrylsäure, Methacrylsäure und/oder eine Methylacrylsäure und/oder einen Alkylester der Acrylsäure umfasst.

17. Urethanacrylat gemäß mindestens einem der Ansprüche 1-16, **dadurch gekennzeichnet, dass** das Di- oder Polyisocyanat Toluoldiisocyanat, Diphenylmethandiisocyanat, Hexamethylendiisocyanat, Isophorondiisocyanat, Tetramethylxyloldiisocyanat, Dicyclohexylmethandiisocyanat, Furandiisocyanat, Tetrahydrofurandiisocyanat, Cyclohexylendiisocyanat, Xyloldiisocyanat, Naphthalendiisocyanat, Phenylendiisocyanat, Nonantriisocyanat und/oder Triphenylmethantriisocyanat.

18. Urethanacrylat gemäß mindestens einem der Ansprüche 1-17, **dadurch gekennzeichnet, dass** die Rohstoffzusammensetzung zusätzlich mindestens eine Carbonsäure umfasst.

19. Urethanacrylat gemäß Anspruch 18, **dadurch gekennzeichnet, dass** die Carbonsäure Octansäure, 2-Ethylhexansäure, 2-Propylheptansäure, Cecansäure und/oder Trimethylhexansäure ist.

20. Urethanacrylat gemäß mindestens einem der Ansprüche 1-19, **dadurch gekennzeichnet, dass** die Komponente (A) ein ethoxyliertes 2,4,8,10-Tetraoxaspiro[5.5]undecan-3,9-diethanol umfasst, das durchschnittlich 3-6 Ethoxyeinheiten/Hydroxylgruppe aufweist, dass die Komponente (B) Hydroxyethylacrylat umfasst und dass die Komponente (C) Isophorondiisocyanat umfasst.

## Revendications

1. Acrylate d'uréthane qui est un produit de réaction d'une composition de matière première comprenant au moins (A) un composant diol, (B) un composant ayant une insaturation acrylique et (C) un composant isocyanate **caractérisé, en ce que** ledit composant (A) comprend au moins un 2,4,8,10-tétraoxaspiro[5.5]undécane-3,9-dialcanol alcoxylé de formule 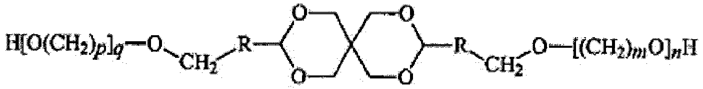 dans lequel chaque substituant R est individuellement un groupe C₁-C₈ alkylène linéaire ou ramifié et *m, n, p* et *q* sont individuellement des entiers étant chacun au moins 1, **en ce que** ledit composant (B) comprend au moins un acrylate d'hydroxyalkyle, un méthacrylate d'hydroxyalkyle et/ou un méthylacrylate d'hydroxyalkyle, et/ou comprend un acide acrylique, un acide méthacrylique et/ou un acide méthylacrylique et/ou un ester alkylique correspondant d'un dit acide acrylique, et **en ce que** ledit composant (C) comprend au moins un diisocyanate ou un polyisocyanate et/ou un dérivé de celui-ci.

2. Acrylate d'uréthane selon la revendication 1, **caractérisé en ce que** ledit hydroxyalkyle est l'hydroxy-C₁-C₁₂-alkyle.

3. Acrylate d'uréthane selon la revendication 1 ou 2, **caractérisé en ce que** ledit composant (B) comprend le (méth)acrylate d'hydroxyéthyle, le (méth)acrylate d'hydroxypropyle, le di(méth)acrylate de triméthylolpropane et/ou le tri(méth)acrylate de pentaérythritol.

4. Acrylate d'uréthane selon l'une quelconque des revendications 1 à 3, **caractérisé en ce que** *m* et *p* sont indépendamment des entiers étant chacun entre 1 et 6.

5. Acrylate d'uréthane selon l'une quelconque des revendications 1 à 3, **caractérisé en ce que** *m* et *p* sont indépendamment des entiers étant chacun entre 1 et 4.

6. Acrylate d'uréthane selon l'une quelconque des revendications 1 à 5, **caractérisé en ce que** *n* et *q* sont indépendamment des entiers étant chacun entre 1 et 10.

7. Acrylate d'uréthane selon l'une quelconque des revendications 1 à 5, **caractérisé en ce que** *n* et *q* sont indépendamment des entiers étant chacun entre 1 et 6.

8. Acrylate d'uréthane selon l'une quelconque des revendications 1 à 6, **caractérisé en ce que** chaque substituant R est un groupe alkyle de formule

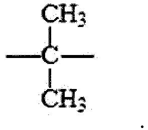

9. Acrylate d'uréthane selon l'une quelconque des revendications 1 à 8, **caractérisé en ce que** ledit 2,4,8,10-tétraoxaspiro[5.5]undécane-3,9-dialcanol alcoxylé est un 2,4,8,10-tétraoxaspiro[5.5]undécane-3,9-diéthanol alcoxylé.

10. Acrylate d'uréthane selon l'une quelconque des revendications 1 à 9, **caractérisé en ce que** ledit alcoxylé signifie éthoxylé, propoxylé et/ou butoxylé.

11. Acrylate d'uréthane selon l'une quelconque des revendications 1 à 10, **caractérisé en ce que** ledit composant (A) comprend 50 à 100 % en poids dudit 2,4,8,10-tétraoxaspiro[5.5]undécane-3,9-dialcanol alcoxylé.

12. Acrylate d'uréthane selon l'une quelconque des revendications 1 à 10, **caractérisé en ce que** ledit composant (A) comprend 70 à 100 % en poids dudit 2,4,8,10-tétraoxaspiro[5.5]undécane-3,9-dialcanol alcoxylé.

13. Acrylate d'uréthane selon l'une quelconque des revendications 1 à 10, **caractérisé en ce que** ledit composant (A) comprend 80 à 100 % en poids dudit 2,4,8,10-tétraoxaspiro[5.5]undécane-3,9-dialcanol alcoxylé.

14. Acrylate d'uréthane selon l'une quelconque des revendications 1 à 13, **caractérisé en ce que** ledit 2,4,8,10-tétraoxaspiro[5.5]undécane-3,9-diéthanol alcoxylé est un 2,4,8,10-tétraoxaspiro[5.5]undécane-3,9-diéthanol éthoxylé et/ou propoxylé.

15. Acrylate d'uréthane selon l'une quelconque des revendications 1 à 14, **caractérisé en ce que** ledit composant (B) comprend 50 à 100 % en poids dudit acrylate d'hydroxyalkyle, méthacrylate d'hydroxyalkyle et/ou méthylacrylate d'hydroxyalkyle.

16. Acrylate d'uréthane selon l'une quelconque des revendications 1 à 15, **caractérisé en ce que** ledit composant (B) comprend 50 à 100 % en poids dudit acide acrylique, acide méthacrylique et/ou d'un acide méthylacrylique et/ou d'un ester alkylique d'un dit acide acrylique.

17. Acrylate d'uréthane selon l'une quelconque des revendications 1 à 16, **caractérisé en ce que** ledit diisocyanate ou polyisocyanate est le diisocyanate de toluène, le diisocyanate de diphényl méthane, le diisocyanate d'hexaméthylène, le diisocyanate d'isophorone, le diisocyanate de tétraméthylxylène, le diisocyanate de dicyclohexyl méthane, le diisocyanate de furane, le diisocyanate de tétrahydrofurane, le diisocyanate de cyclohexylène, le diisocyanate de xylène, le diisocyanate de naphtalène, le diisocyanate de phénylène, le triisocyanate de nonane et/ou le triisocyanate de triphényl méthane.

18. Acrylate d'uréthane selon l'une quelconque des revendications 1 à 17, **caractérisé en ce que** ladite composition de matière première comprend au moins un acide carboxylique.

19. Acrylate d'uréthane selon la revendication 18, **caractérisé en ce que** ledit acide carboxylique est l'acide octanoïque, l'acide 2-éthylhexanoïque, l'acide 2-propylheptanoïque, l'acide cécanoïque et/ou l'acide triméthylhexanoïque.

20. Acrylate d'uréthane selon l'une quelconque des revendications 1 à 19, **caractérisé en ce que** ledit composant (A) comprend un 2,4,8,10-tétraoxaspiro[5.5]undécane-3,9-diéthanol éthoxylé ayant une moyenne de 3 à 6 motifs éthoxy/groupe hydroxyle, **en ce que** ledit composant (B) comprend l'acrylate d'hydroxyéthyle et **en ce que** ledit composant (C) comprend le diisocyanate d'isophorone.
